# EUROPEAN PATENT SPECIFICATION

(11) **EP 1 988 812 B1**
(45) Date of publication and mention of the grant of the patent: **21.11.2012**
(21) Application number: 07763368.3
(22) Date of filing: 06.02.2007
(51) Int. Cl.: A61M 25/01, A61B 1/05, A61B 1/005, A61B 1/06

(54) **ENDOSCOPE WITH AN IMAGING CATHETER ASSEMBLY AND METHOD OF CONFIGURING AN ENDOSCOPE**
ENDOSKOP MIT EINER BILDGEBENDEN KATHETERANORDNUNG UND VERFAHREN ZUR EINSTELLUNG EINES ENDOSKOPS
ENDOSCOPE À ENSEMBLE CATHÉTER D'IMAGERIE ET PROCÉDÉ DE CONFIGURATION D'UN ENDOSCOPE

(30) Priority: 06.02.2006 US 771099 P; 12.12.2006 US 609838; 23.01.2007 US 626189
(43) Date of publication of application: 12.11.2008
(73) Proprietor: Avantis Medical Systems, Inc., Sunnyvale, CA 94085-4511 (US)
(72) Inventor: BAYER, Lex, Palo Alto, CA 94306 (US); DESAI, Rupesh, San Jose, California 95132 (US); HIGGINS, John, Los Altos, California 94023 (US)
(74) Representative: ZBM Patents
(86) International application number: PCT/US2007/003322
(87) International publication number: WO 2007/092533

(56) References cited:
- US-A- 5 679 216
- US-A1- 2005 272 977
- DATABASE WPI Week 200564 Derwent Publications Ltd., London, GB; AN 2005-620837 XP002443813 & CN 1 628 603 A (TIAN D) 22 June 2005 (2005-06-22)

## Description

### TECHNICAL FIELD OF THE INVENTION

The present invention relates to an endoscope, an imaging catheter assembly, and a method of configuring an endoscope.

### BACKGROUND OF THE INVENTION

An endoscope is a medical device comprising a flexible tube and a camera mounted on the distal end of the tube. The endoscope is insertable into an internal body cavity through a body orifice or a surgical incision to examine the body cavity and tissues for diagnosis. The tube of the endoscope has one or more longitudinal channels, through which an instrument can reach the body cavity to take samples of suspicious tissues or to perform other surgical procedures such as polypectomy.

There are many types of endoscopes, and they are named in relation to the organs or areas with which they are used. For example, gastroscopes are used for examination and treatment of the esophagus, stomach and duodenum; colonoscopes for the colon; bronchoscopes for the bronchi; laparoscopes for the peritoneal cavity; sigmoidoscopes for the rectum and the sigmoid colon; arthroscopes for joints; cystoscopes for the urinary bladder; and angioscopes for the examination of blood vessels.

Conventional endoscopes are characterized by a single forward viewing camera mounted at the distal end of the endoscope to transmit an image to an eyepiece or video display at the proximal end. The camera is used to assist a medical professional in advancing the endoscope into a body cavity and looking for abnormalities. The camera provides the medical professional with a two-dimensional view from the distal end of the endoscope. To capture an image from a different angle or in a different portion, the endoscope must be repositioned or moved back and forth. Repositioning and movement of the endoscope prolongs the procedure and causes added discomfort, complications, and risks to the patient. Additionally, in an environment such as the lower gastro-intestinal tract, flexures, tissue folds and unusual geometries of the organ may prevent the endoscope's camera from viewing all areas of the organ. The inability to view an area may cause a potentially malignant (cancerous) polyp to be missed.

This problem can be overcome by providing an auxiliary camera, which presents an image from a different point-of-view and enables viewing of areas not viewable by the endoscope's main camera. The auxiliary camera can be oriented backwards to face the main camera. This arrangement of cameras can provide both front and rear views of an area or an abnormality. In the case of polypectomy where a polyp is excised by placing a wire loop around the base of the polyp, the camera arrangement allows better placement of the wire loop to minimize damage to the adjacent healthy tissue.

An imaging assembly according to the preamble of claim 1 is known from CN 1628603.

### SUMMARY OF THE INVENTION

In accordance with one aspect of the invention, an assembly includes a tubular body, an imaging device, and a shape memory link that connects the imaging device to the tubular body, as defined in the appended claims.

The shape memory link has a U-shaped natural configuration with two end segments. Preferably, one of the end segments is connected to the imaging device and the other end segment is connected to the tubular body. The end segments may be substantially parallel to each other.

The shape memory link preferably returns to its natural configuration after a force that straightens the shape memory link is removed.

The imaging device may have a cylindrical housing having first and second ends and an imaging unit disposed in the housing. The imaging unit may be disposed on the first end of the housing, and the link may be connected to the second end of the housing.

The shape memory link may include a shape memory element and flexible electrical conductors connecting electrical conductors from the imaging device to electrical conductors in the tubular body. The flexible electrical conductors preferably are a flexible PCB. The shape memory element and flexible PCB may be placed side by side in a width direction of the flexible PCB, attached to each other, or stacked to form a layered structure. The electrical conductors in the tubular body can be embedded in a wall of the tubular body or disposed in a channel of the tubular body.

The assembly may further include a light source that is placed on the link. The light source may be placed on a curved section of the link, more specifically on a concaved side of a curve section of the link. The light source can be centered in a U-shape section of the link. The light source can also be placed in a center of an S-shaped section of the link and faces away from the tubular body. Preferably, the light source faces the distal end of the tubular body. In some embodiment, the assembly may include a plurality of light sources placed on the link.

In accordance with another aspect of the invention, an endoscope assembly includes an insertion tube having an end and an imaging catheter assembly. The imaging catheter assembly may have a tubular body, a first imaging device, and a shape memory link that connects the first imaging device to the tubular body.

The endoscope assembly may include a second imaging device and a second light source, both being positioned on the end of the insertion tube. The first and second imaging devices preferably provide adjacent or overlapping viewing areas. More preferably, the first and second imaging devices provide different views of a same area. In some embodiment, the first imaging device and first light source and the second imaging device and second light source are turned on and off alternately. Preferably, the first imaging device and first light source and the second imaging device and second light source are turned on and off at a sufficiently high frequency such that eyes do not sense that they are being intermittently turned on and off.

Furthermore, each of the imaging devices may be covered by a first polarizer filter, and each of the opposing light sources may be covered by a second polarizer filter orientated at 90° relative to the opposing first polarizer filter. In some embodiment, only one of the imaging devices is covered by a first polarizer filter, and only the light source opposing the only one of the imaging devices is covered by a second polarizer filter orientated at 90° relative to the first polarizer filter.

In accordance with still another aspect of the invention, a method of configuring an endoscope includes the steps of straightening a U-shaped flexible shape memory link at a distal end of an imaging catheter assembly, inserting the straightened distal end of the imaging catheter assembly into a channel of an endoscope's insertion tube from a proximal end of the insertion tube; and pushing the imaging catheter assembly further into the insertion tube until an auxiliary imaging device 42 and the flexible link 44 are pushed out of the distal end of the insertion tube so that the flexible link returns to its natural bent configuration.

In accordance with yet another aspect of the invention, a minor endoscope adapted to be inserted into and through a channel of a major endoscope includes an elongated body, an imaging device located proximate to one end, and a flexible shape memory material coupled to the imaging device to the body, wherein the flexible shape memory material may be straightened for insertion into the major endoscope and wherein it assumes its original shape when it is extended beyond the major endoscope, the imaging device providing a retrograde view when the flexible shape memory material assumes its original shape.

In accordance with a further aspect of the invention, a minor endoscope adapted to be inserted into and through a channel of a major endoscope includes an elongated body, an imaging device located proximate to one end, and a channel for accessories disposed along its length.

In accordance with a still further aspect of the invention, a minor endoscope adapted to be inserted into and through a channel of a major endoscope includes an elongated body, an imaging device located proximate to one end, and a steering mechanism allowing the end minor endoscope to be turned within a dimension approximate to the diameter of the major endoscope when it is extended beyond the major endoscope permitting the imaging device to provide a retrograde view.

In accordance with a yet further aspect of the invention, a minor endoscope to be inserted into and through a channel of a major endoscope is positioned to extend beyond the major endoscope, and permits the simultaneous imaging of a common region of the body from a substantially forward viewing angle from the major endoscope and a substantially retrograde viewing angle from the minor endoscope.

In accordance with a still yet further aspect of the invention, a minor endoscope minor endoscope to be inserted into and through a channel of a major endoscope is positioned to extend beyond the major endoscope, and permits the simultaneous imagine of a common region of the body from two different perspectives, one provided by the major endoscopes, the second provided by the minor endsocope.

In accordance with another aspect of the invention, an endoscope assembly includes an insertion tube having an end, a first imaging device mounted on the end of the insertion tube; and an imaging catheter assembly. The imaging catheter assembly preferably includes an end and a second imaging device mounted on the end of the imaging catheter assembly. The imaging catheter assembly may be partially disposed inside the insertion tube. The second imaging device may extend beyond the end of the insertion tube. The first and second imaging devices may face each other.

In accordance with another aspect of the invention, an endoscopic assembly includes an endoscope having a proximal end, a distal end, and a channel extending between the proximal and distal ends; a first imaging sensor disposed on the distal end of the endoscope, the first imaging sensor providing a forward-looking field of view; an imaging catheter assembly disposed in the channel, a distal region of the imaging catheter assembly configured to form a U-shape upon deployment beyond the distal end of the endoscope; and a second imaging sensor disposed on the distal end of the imaging catheter assembly, the second imaging sensor providing a rearward-looking field of view.

In some embodiments, the imaging catheter assembly is manufactured from a shape-memory alloy causing the imaging catheter assembly to configure from a substantially elongated shape to the U-shape upon deployment beyond the distal end of the endoscope. In still other embodiments, the forward-looking field of view and the rearward looking field of view have adjacent or overlapping edges.

### BRIEF DESCRIPTION OF THE DRAWINGS

Figure 1 shows a perspective view of an endoscope with an imaging catheter assembly according to one embodiment of the present invention.
Figure 2 shows a perspective view of the imaging catheter assembly shown in Figure 1.
Figure 3 shows a perspective view of the distal end of the endoscope of Figure 1.
Figure 4 shows a perspective view of a portion of a link belonging to the imaging catheter assembly shown in Figure 2.
Figure 5 shows an exploded perspective view of the link belonging to the imaging catheter assembly of Figure 2.
Figure 6 shows a perspective view of an endoscope with an imaging catheter assembly according to another embodiment of the present invention.
Figure 7 shows a perspective view of an imaging catheter assembly according to still another embodiment of the present invention.
Figure 8 shows a perspective view of an imaging catheter assembly according to yet another embodiment of the present invention.
Figure 9 shows a perspective view of an imaging catheter assembly according to a further embodiment of the present invention.
Figure 10 shows a perspective view of an imaging catheter assembly according to a yet further embodiment of the present invention.

### DETAILED DESCRIPTION OF EMBODIMENTS OF THE INVENTION

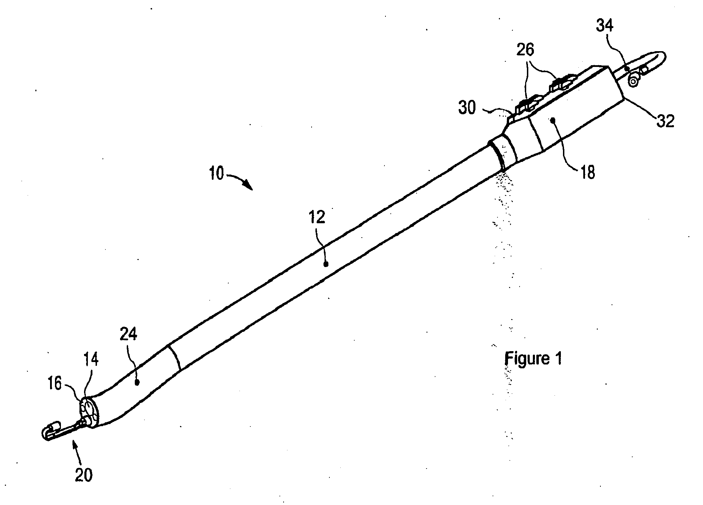

Figure 1 illustrates a first exemplary endoscope 10 of the present invention. This endoscope 10 can be used in a variety of medical procedures in which imaging of a body tissue, organ, cavity or lumen is required. The types of procedures include, for example, anoscopy, arthroscopy, bronchoscopy, colonoscopy, cystoscopy, EGD, laparoscopy, and sigmoidoscopy.

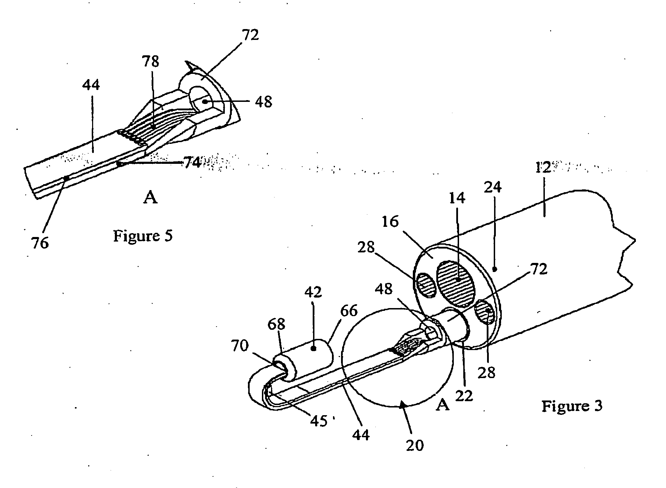

The endoscope 10 of Figure 1 includes an insertion tube 12, a main imaging device 14 disposed at the distal end 16 of the insertion tube 12 (Figure 3), a control handle 18 connected to the proximal end of the insertion tube 12, and an imaging catheter assembly 20 disposed at the distal end 16 of the insertion tube 12 and inside the insertion tube 12.

The insertion tube 12 may be detachable from the control handle 18 or may be integrally formed with the control handle 18. The diameter, length and flexibility of the insertion tube 12 depend on the procedure for which the endoscope 10 is used.

In the illustrated embodiment, as shown in Figure 3, the insertion tube 12 has one longitudinal channel 22 for accommodating the imaging catheter assembly 20. In general, however, the insertion tube 12 may have more than one longitudinal channel through which an instrument can reach the body cavity to perform any desired procedures, such as to take samples of suspicious tissues or to perform other surgical procedures such as polypectomy. The instruments may be, for example, a retractable needle for drug injection, hydraulically actuated scissors, clamps, grasping tools, electrocoagulation systems, ultrasound transducers, electrical sensors, heating elements, laser mechanisms and other ablation means. In some embodiments, one of the channels can be used to supply a washing liquid such as water for washing. A cap (not shown) may be included at the opening of the washing channel to divert the washing liquid onto a lens of the main imaging device 14 for cleaning. Another or the same channel may be used to supply a gas, such as CO₂ or air, into the organ. The channels may also be used to extract fluids or inject fluids, such as a drug in a liquid carrier, into the body. Various biopsy, drug delivery, and other diagnostic and therapeutic devices may also be inserted via the channels to perform specific functions.

The insertion tube 12 preferably is steerable or has a steerable distal end region 24 as shown in Figure 1. The length of the distal end region 24 may be any suitable fraction of the length of the insertion tube 12, such as one half, one third, one fourth, one sixth, one tenth, or one twentieth. The insertion tube 12 may have control cables (not shown) for the manipulation of the insertion tube 12. Preferably, the control cables are symmetrically positioned within the insertion tube 12 and extend along the length of the insertion tube 12. The control cables may be anchored at or near the distal end 16 of the insertion tube 12. Each of the control cables may be a Bowden cable, which includes a wire contained in a flexible overlying hollow tube. The wires of the Bowden cables are attached to controls 26 in the handle 18. Using the controls, the wires can be pulled to bend the distal end region 24 of the insertion tube 12 in a given direction. The Bowden cables can be used to articulate the distal end region 24 of the insertion tube 12 in different directions.

The main imaging device 14 at the distal end 16 of the insertion tube 12 may include, for example, a lens, single chip sensor, multiple chip sensor or fiber optic implemented devices. The main imaging device 14, in electrical communication with a processor and/or monitor, may provide still images or recorded or live video images. In addition to the main imaging device 14, the distal end 16 of the insertion tube 12 may include one or more light sources 28 (Figure 3), such as light emitting diodes (LEDs) or fiber optical delivery of light from an external light source. The light sources 28 preferably are equidistant from the main imaging device 14 to provide even illumination. The intensity of each light source 28 can be adjusted to achieve optimum imaging. The circuits for the main imaging device 14 and light sources 28 may be incorporated into a printed circuit board (PCB).

The insertion tube 12 may include a flexible ribbon coil (not shown) and a flexible sheath (not shown) that is used to protect the internal components of the insertion tube 12, such as the channels, wires and cables, from the environment of the body.

Preferably, the control handle 18 has one or more ports and/or valves (not shown) for controlling access to the channels of the insertion tube 12. The ports and/or valves can be air or water valves, suction valves, instrumentation ports, and suction/instrumentation ports. As shown in Figure 1, the control handle 18 may additionally include buttons 30 for taking pictures with the main imaging device 14, the imaging catheter assembly 20, or both.

The proximal end 32 of the control handle 18 may include an accessory outlet 34 (Figure 1) that provides fluid communication between the air, water and suction channels and the pumps and related accessories. The same outlet or a different outlet can be used for electrical lines to light and imaging components at the distal end of the endoscope 10.

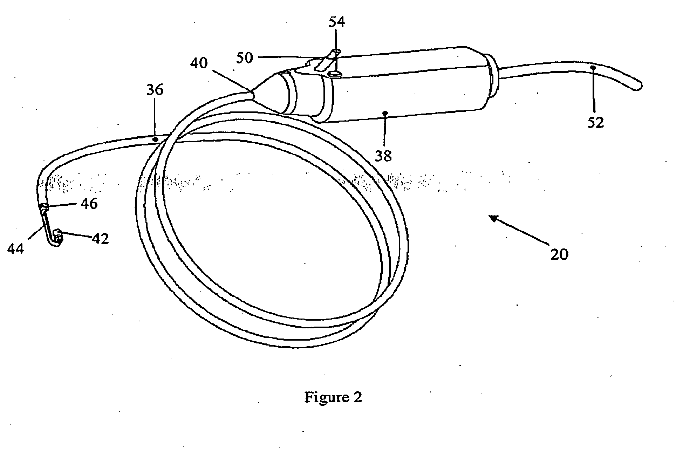

As shown in Figure 2, the imaging catheter assembly 20 may include a tubular body 36, a handle 38 connected to the proximal end 40 of the tubular body 36, an auxiliary imaging device 42, a link 44 that provides physical and/or electrical connection between the auxiliary imaging device 42 to the distal end 46 of the tubular body 36, and a light source 45 (illustrated in Figure 3).

The imaging catheter assembly 20 is used to provide an auxiliary imaging device at the distal end of the endoscope 10. To this end, the imaging catheter assembly 20 is placed inside the channel 22 of the endoscope's insertion tube 12 with its auxiliary imaging device 42 disposed beyond the distal end 16 of the insertion tube 12. This can be accomplished by first inserting the distal end of the imaging catheter assembly 20 into the insertion tube's channel 22 from the endoscope's handle 18 and then pushing the imaging catheter assembly 20 further into the channel 22 until the auxiliary imaging device 42 and link 44 of the imaging catheter assembly 20 are positioned outside the distal end 16 of the insertion tube 12 as shown in Figure 3.

The tubular body 36 of the imaging catheter assembly 20 may have any suitable configuration. In terms of its length, the tubular body 36 preferably is sufficiently long such that the auxiliary imaging device 42 and link 44 can extend beyond the distal end 16 of the insertion tube 12. The preferred cross-section of the illustrated tubular body 36 is circular, although the cross-section may have any other suitable configuration, such as an elliptical or polygonal configuration.

In the illustrated embodiment, as shown in Figure 3, the tubular body 36 has a channel 48 expanding its entire length, although the tubular body 36 generally may have no channels or two or more channels. This channel 48 may be used for various purposes. For example, the channel 48 may be used for passing instruments, such as snares or biopsy forceps, from the proximal end of the imaging catheter assembly 20 to the distal end. Each of the instruments may be incorporated into the imaging catheter assembly 20, rather than as a separate instrument. The opening for the channel 48 at the distal end may include a slope or ramp at a predetermined angle so as to guide any instruments away from the link 44 and into a predefined position and alignment so as to be within the field-of view and focus of the imaging catheter assembly 20.

The channel 48 may also be used to control the flow of fluid into and from the body cavity. For example, the channel 48 may be used to control the flow of air into and from the body cavity (suction or insufflation) as well as to supply water to, for example, wash the auxiliary imaging device 42. The channel 48 may further be used for the routing of electrical conductors between the auxiliary imaging device 42 and the handle 38. The channel 48 can be provided with a lubricious liner to ease the movement of an instrument inside the channel 48. The lubricious liner may be made from any suitable material such as PTFE or Polyimide.

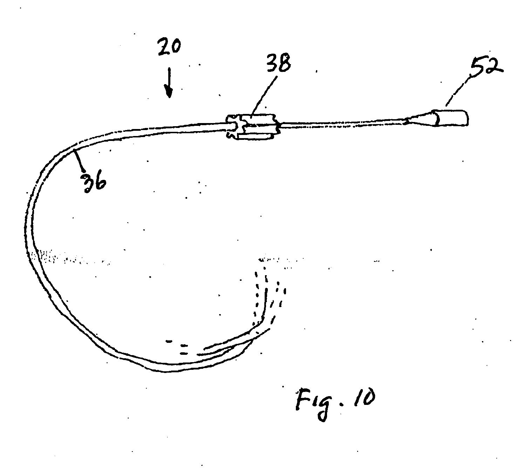

The handle 38 of the imaging catheter assembly 20 may control various functions of the imaging catheter assembly 20. For example, the handle 38 may serve as a convenient way to deploy and/or rotate the imaging catheter assembly 20 inside the channel 22 of the insertion tube 12. The handle 38 may also provide an access port 50 for the channel 48 of the tubular body 36. The handle 38 may additionally provide a connector 52, to which electrical conductors from the auxiliary imaging device 42 and other components of the imaging catheter assembly 20 are connected. The connector 52 can be used to connect the auxiliary imaging device 42 and other components to a device outside of the imaging catheter assembly 20, such as a control box. The handle 38 may further provide a switch 54 that is used to operate the auxiliary imaging device 42 to capture still images. Alternatively, as shown in Figure 10, the handle 38 may be simply configured to be used to rotate the imaging catheter assembly 20.

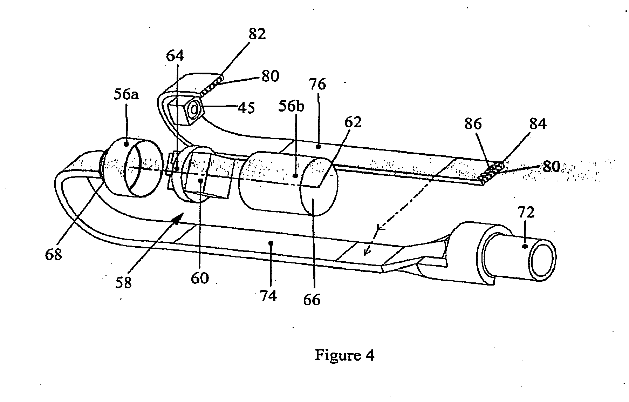

As shown in Figure 4, the auxiliary imaging device 42 may include a housing 56a, 56b and an imaging unit 58 disposed in the housing 56a, 56b. In this embodiment, the housing 56a, 56b has a generally cylindrical configuration, but in general the housing may have any suitable configuration such as a spherical or cubic configuration. The housing 56a, 56b includes two parts 56a, 56b that are sealingly joined to form the housing 56a, 56b. The housing 56a, 56b may be made from any suitable material such as stainless steel or a plastic material.

As shown in Figure 4, the imaging unit 58 may include a lens 62, an imaging sensor 60, and a printed circuit board (PCB) 64 containing electrical components of the imaging unit 58. The lens 62 is installed in an aperture on a first end 66 of the housing 56a, 56b, and may include a plurality of optical elements in a holder or barrel which focuses the incoming light from the surroundings onto a photosensitive area of the image sensor 60.

The imaging sensor 60 may be an electronic device which converts light incident on photosensitive semiconductor elements into electrical signals. The imaging sensor 60 may detect either color or black-and-white images. The signals from the imaging sensor 60 can be digitized and used to reproduce an image that is incident on the imaging sensor 60. Two commonly used types of image sensors are Charge Coupled Devices (CCD) such as a VCC-5774 produced by Sanyo of Osaka, Japan and Complementary Metal Oxide Semiconductor (CMOS) camera chips such as an OVT 6910 produced by Omni Vision of Sunnyvale, California.

Alternatively, the imaging unit 58 may include a coherent fiber optic bundle and a lens for channeling light into the coherent fiber optic bundle, which then delivers the light from the distal end of the imaging catheter assembly 20 to an imaging sensor located at the proximal end of, or external to, the imaging catheter.

On its second end 68, the housing 56a, 56b of the auxiliary imaging device 42 may include an opening 70 (Figure 3) for a flexible PCB 76 (Figure 4) to pass through for connection with the imaging unit 58. The flexible PCB 76 electrically connects the imaging unit 58 to the electrical conductors 78 (Figure 5) which extend through tubular body 36.

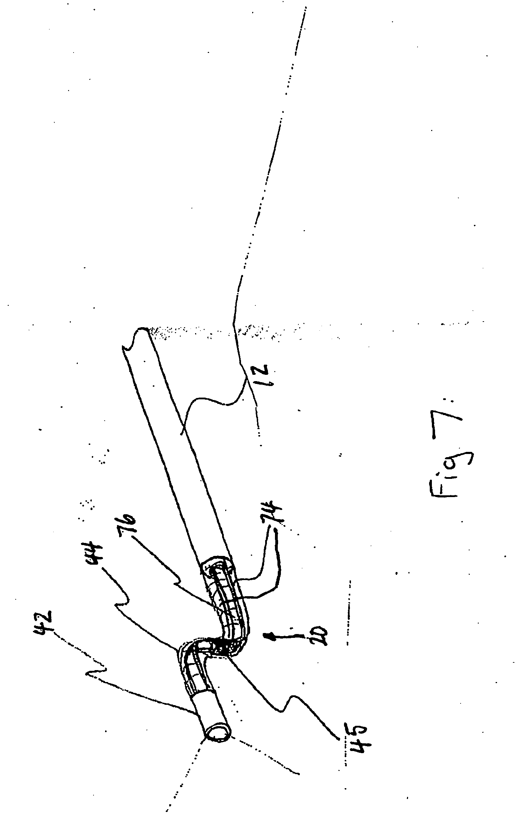

When the imaging catheter assembly 20 is properly installed in the insertion tube 12, the auxiliary imaging device 42 of the imaging catheter assembly 20 preferably faces backwards towards the main imaging device 14 as illustrated in Figure 3. The auxiliary imaging device 42 may be oriented so that the auxiliary imaging device 42 and the main imaging device 14 have adjacent or overlapping viewing areas. Alternatively, the auxiliary imaging device 42 may be oriented so that the auxiliary imaging device 42 and the main imaging device 14 simultaneously provide different views of the same area. Preferably, the auxiliary imaging device 42 provides a retrograde view of the area, while the main imaging device 14 provides a front view of the area. However, the auxiliary imaging device 42 could be oriented in other directions to provide other views, including a forward view, as shown in Figure 7, and views that are substantially parallel to the axis of the main imaging device 14. The light source 45 of the auxiliary imaging device may also face forward as shown in Figure 7. Such an embodiment could be useful when the size of the imaging catheter assembly permits to reach locations that are too narrow to allow passage of the insertion tube.

As shown in Figures 2 and 3, the link 44 connects the auxiliary imaging device 42 to the distal end 46 of the tubular body 36. The link 44 is a flexible link that is at least partially made from a flexible shape memory material that substantially tends to return to its original shape after deformation. Shape memory materials are well known and include shape memory alloys and shape memory polymers. A suitable flexible shape memory material is a shape memory alloy such as nitinol. The flexible link 44 is straightened to allow the distal end of the imaging catheter assembly 20 to be inserted into the proximal end of channel 22 of the insertion tube 12 and then pushed towards the distal end 16 of the insertion tube 12. When the flexible link 44 is straightened inside the channel 22 of the insertion tube 12, the first end 66 of the auxiliary imaging device 42 faces away from the tubular body 36, a direction parallel to the main imaging device 14, while the second end 68 of the auxiliary imaging device 42 faces back towards the tubular body 36 and handle 38. When the auxiliary imaging device 42 and flexible link 44 are pushed sufficiently out of the distal end 16 of the insertion tube 12, the flexible link 44 resumes its natural bent configuration as shown in Figure 3. The natural configuration of the flexible link 44 is the configuration of the flexible link 44 when the flexible link 44 is not subject to any force or stress. When the flexible link 44 resumes its natural bent configuration, the first end 66 of the auxiliary imaging device 42 faces substantially back towards the tubular body 36 (Figure 2) and back towards the distal end 16 of the insertion tube 12 (Figure 3) while the second end 68 of the auxiliary imaging device 42 faces away from the tubular body 36 (Figure 2) and away from the distal end 16 of the insertion tube 12 (Figure 3).

The flexible link may have any suitable configuration that allows it to be straightened under force and to return to its natural bent configuration when the force is removed. For example, the flexible link may have a U-shaped, S-shaped, right angle, or ramp configuration. In the illustrated embodiment, the flexible link 44 has a U-shaped natural configuration with two end segments that are substantially parallel to each other. Preferably, the distance between the end segments is equal to or less than a diameter of the insertion tube. One of the end segments is connected to the auxiliary imaging device 42 and other end segment is connected to the tubular body 36. Although the end segment connected to the tubular body 36 is much longer in the illustrated embodiment, the end segment connected to the auxiliary imaging device 42 may be longer in other embodiments. The flexible link 44 may have a generally elongated flat configuration with a hollow tubular end 72 for connection to the tubular body 36. As shown in Figure 4, the hollow tubular end 72 of the flexible link 44 may be attached to the distal end 46 of the tubular body 36 by concentrically mating with the channel 48 of the tubular body 36. The attachment may be accomplished by any suitable means including adhesive bonding, welding or soldering. At the other end, the flexible link 44 may be joined to the auxiliary imaging device 42 by any suitable means such as adhesive bonding, welding or soldering.

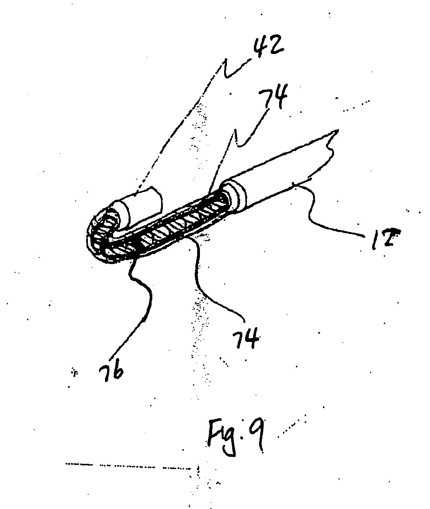

In the illustrated embodiment, as shown in Figures 4 and 5, the flexible link 44 may include a flexible shape memory element 74 and a flexible PCB 76 that electrically connects the auxiliary imaging device 42 to the electrical conductors 78 in the tubular body 36. The flexible shape memory element 74 preferably performs the shape memory function of the flexible link 44, and the flexible PCB 76 is attached to the flexible shape memory element 74 so that its shape changes with the shape of the flexible shape memory element 74. Alternatively, the flexible PCB 76 and flexible shape memory element 74 may be merely placed next to one another but not attached. Even when the flexible PCB 76 and flexible shape memory element 74 are not attached to each other, they will still undergo substantially the same shape changes as long as they are appropriately configured (such as if their lengths are similar). In the illustrated embodiment, the flexible shape memory element 74 and flexible PCB 76 have a similar configuration and are stacked in the thickness direction of the flexible PCB 76 to form a layered structure. In general, however, they may have different configurations and may be arranged relative to each other in any other suitable manner. For example, one of the shape memory element and flexible PCB may be wider than the other, and they may be placed side by side in the width direction of the flexible PCB 76, as shown in Figure 9 as opposed to being stacked in the thickness direction of the flexible PCB 76. Furthermore, the shape memory element may include two components, and the flexible PCB 76 may be placed between the two components in either the thickness direction or the width direction of the flexible PCB 76. In a preferred embodiment, the components of the shape memory element may each be a wire-shaped shape memory component, and the wire-shaped components may be placed on the two width sides of the flexible PCB, respectively. This symmetrical arrangement provides a common central bending axis for both the shape memory element and the flexible PCB.

As shown in Figure 4, the flexible PCB 76 includes electrical conductors 80 that connect the auxiliary imaging device 42 to the electrical conductors 78 in the tubular body 36. At one end 82 of the flexible PCB 76, the electrical conductors 80 of the flexible PCB 76 are connected to the auxiliary imaging device 42. At the other end 84 of the flexible PCB 76, the electrical conductors 80 of the flexible PCB 76 are connected to the electrical conductors 78 in the tubular body 36. This end 84 of the flexible PCB 76 may have pads 86 for the connection between the electrical conductors 78 and electrical conductors 80.

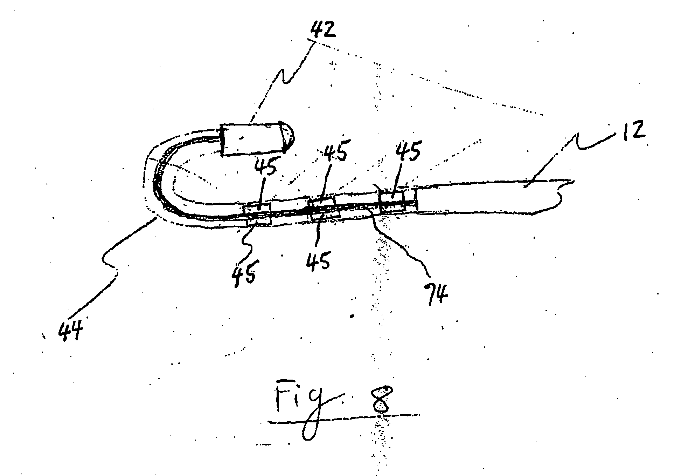

In the illustrated embodiment, the light source 45 (as well as other components) of the imaging catheter assembly 20 is placed on the flexible link 44, in particular on the curved concave portion of the flexible link 44. Multiple light sources 45 may be located along the flexible link 44, including along a straight portion of the flexible link 44 as shown in Figure 8. The light sources 45 provide illumination for the auxiliary imaging device 42 and may face substantially the same direction as the auxiliary imaging device 42 as shown in Fig 4 or perpendicular to it as shown in Fig 8. The light sources 45 may be light emitting diodes (LEDs) soldered onto conductive pads (not shown) on the flexible PCB 76. Alternatively, fiber optic bundles may be used to deliver light from an external light source to the distal region of the imaging catheter.

The flexible link may be encapsulated or shrouded by flexible tubing, heat-shrinkable tubing, urethanes, rubber or silicon so as to allow smooth profile transition from the tubular body to the imaging device. This encapsulation may be translucent to allow light from the light source to project through the encapsulation, or the encapsulation may include a window section around each light source.

Since the main imaging device 14 and its light source 28 face the auxiliary imaging device 42 and its light source 45, the light sources 28, 45 of the imaging devices 14, 42 may interfere with the opposing imaging device 42, 14. That is, light source 28 may shine directly into auxiliary imaging device 42 and light source 45 may shine directly into main imaging device 14, degrading both images. To reduce the interference, polarizer filters may be used with the imaging devices 14, 42 and light sources 28, 45. Specifically, the main imaging device 14 and/or its light source 28 may be covered by a first set of polarizer filters of a given orientation. And the auxiliary imaging device 42 and/or its light source 45 may be covered by a second set of polarizer filters orientated at 90° relative to the first set of polarizer filters. The use of polarizer filters to reduce light interference is well known and will not be described in further detail.

As an alternative to polarizer filters, the imaging devices 14, 42 and their light sources 28, 45 may be turned on and off alternately to reduce or prevent light interference. In other words, when the main imaging device 14 and its light sources 28 are turned on, the auxiliary imaging device 42 and its light source 45 are turned off. And when the main imaging device 14 and its light sources 28 are turned off, the auxiliary imaging device 42 and its light source 45 are turned on. Preferably, the imaging devices 14, 42 and their light sources 28, 45 are turned on and off at a sufficiently high frequency that eyes do not sense that the light sources are being turned on and off.

The auxiliary imaging device 42 and its light source 45 are connected to a control box (not shown) via electrical conductors that extend from the imaging device 42 and light source 45; through the flexible PCB 76, tubular body 36, and handle 38; to the control box. The electrical conductors may carry power and control commands to the auxiliary imaging device 42 and its light source 45 and image signals from the auxiliary imaging device 42 to the control box. In the illustrated embodiment, the electrical conductors 78 in the tubular body 36 may be embedded in the wall of the tubular body 36, or simply in the tubular body if the tubular body does not have a channel, during the fabrication process or disposed in the channel 48 of the tubular body 36. The embedding of the electrical conductors in the tubular body 36 may be done by a braiding or coiling process to achieve the desired stiffness of the tubular body 36. A short length of the embedded electrical conductors may be exposed at either end of the tubular body 36 to allow connections to be made. The connections may then be sealed by means of, for example, heat-shrinking tubing, a sheath or an adhesive.

The control box includes at least an image and signal processing device and a housing in which the image and signal processing device is disposed, although the control box can be configured in any suitable manner. The housing may include a control panel and connectors. The control panel includes buttons and knobs for controlling the functionalities of the control box.

The image and signal processing device may include one or more integrated circuits and memory devices along with associated discrete components. The device allows image signals from the imaging devices 14, 42 to be processed for the enhancement of image quality, extraction of still images from the image signals, and conversion of video format for compatibility with the display device.

The control box preferably processes the video image signal from the auxiliary imaging device 42 and transmits it to a display device such as a television or a monitor such as a liquid crystal display monitor. Still images can be captured from the video image signal using the switch 54 on the handle 38 of the imaging catheter assembly 20. The video image or still image may be displayed on the display device. The display device may also include textual data that are used to display information such as patient information, reference numbers, date, and/or time.

The image signal from the main imaging device 14 may also be processed by the control box in the same way that the image signal from the auxiliary imaging device 42 is processed. The images from the main and auxiliary imaging devices 14, 42 may be displayed on two separate monitors or on the same monitor with a split screen.

The control box may further be used to adjust the parameters of the imaging devices 14, 42 and their light sources 28, 45, such as brightness, exposure time and mode settings. The adjustment can be done by writing digital commands to specific registers controlling the parameters. The registers can be addressed by their unique addresses, and digital commands can be read from and written to the registers to change the various parameters. The control box can change the register values by transmitting data commands to the registers.

The control box may additionally be used as an interface to the patient records database. A large number of medical facilities now make use of electronic medical records. During the procedure relevant video and image data may need to be recorded in the patient electronic medical records (EMR) file. The signal processing circuit can convert image and video data to a format suitable for filing in the patient EMR file such as images in jpeg,.tif, or .bmp format among others. The processed signal can be transmitted to the medical professional's computer or the medical facilities server via a cable or dedicated wireless link. A switch on the control panel can be used to enable this transmission. Alternatively the data can be stored with a unique identification for the patient in electronic memory provided in the control box itself. The signal processing circuit can be utilized to convert the video and image data to be compatible with the electronic medical records system used by the medical professional. The processing may include compression of the data. A cable or a wireless link may be used to transmit the data to a computer.

During endoscopy, a physician may straighten the flexible link 44 of the imaging catheter assembly 20 and then insert the straightened distal end of the imaging catheter assembly 20 into the channel 22 of the endoscope's insertion tube 12 from the handle 18. The imaging catheter assembly 20 can then be pushed towards the distal end 16 of the insertion tube 12. When the auxiliary imaging device 42 and flexible link 44 are pushed out of the distal end 16 of the insertion tube 12, the flexible link 44 resumes its natural bent configuration as shown in Figure 2.

The main imaging device 14 now captures a front-viewing image, and the auxiliary imaging device 42 simultaneously captures a rear-viewing image of the same area. The control box processes the video image signals and transmits them to a display device or display devices for viewing by the physician. The physician can adjust the view of the auxiliary imaging device 42 by rotating the handle 38 of the imaging catheter assembly 20 and/or by pushing or pulling the imaging catheter assembly 20 in the channel 22 of the insertion tube 12. As a result, the physician can inspect a lesion such as a cancer or polyp at various angles.

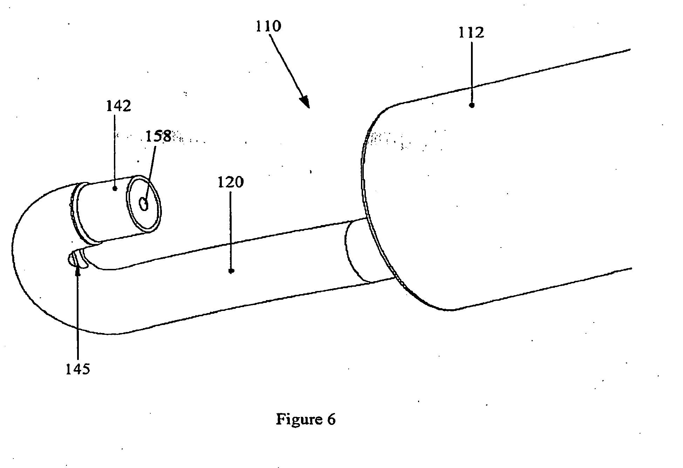

Figure 6 illustrates a further embodiment of the present invention. In this embodiment, the endoscope 110 has an insertion tube 112 and an imaging catheter assembly 120 positioned at the distal end of and inside the insertion tube 112. The imaging catheter assembly 120 includes an auxiliary imaging device 142 disposed at the distal end of the imaging catheter assembly 120. The auxiliary imaging device 142 includes an imaging unit 158 and a light source 145. When the imaging catheter assembly 120 is properly installed in the insertion tube 12, the auxiliary imaging device 142 of the imaging catheter assembly 20 preferably faces backwards towards the main imaging device (not shown). The auxiliary imaging device 142 may be oriented so that the auxiliary imaging device 142 and the main imaging device have adjacent or overlapping viewing areas. Alternatively, the auxiliary imaging device 142 may be oriented so that the auxiliary imaging device 142 and the main imaging device simultaneously provide different views of the same area. Preferably, the auxiliary imaging device 142 provides a retrograde view of the area, while the main imaging device provides a front view of the area. However, the auxiliary imaging device 142 could be oriented in other directions to provide other views, including views that are substantially parallel to the axis of the main imaging device.

The distal end region of the imaging catheter assembly 120 preferably is made by shape setting of the catheter assembly 120 itself. This process is widely used and understood in the art and involves a process combination of heat and fixturing to create the pre-shaped distal end. The pre-shaped distal end may be supported by a piece of a shape memory material such as nitinol set in a similar shape. The imaging catheter assembly 120 may also include a light source 145. In general, this endoscope 110 is similar to the endoscope 10 shown in Figures 1-5, except the distal end portion of the imaging catheter assembly 120.

In an additional embodiment of the present invention, the auxiliary imaging device includes a wireless transceiver, associated circuitry and a battery. The wireless transceiver is configured to receive video signals from the imaging unit of the auxiliary imaging device and to transmit them wirelessly to a control box. Alternatively, the wireless circuit may be implemented in a flexible PCB or the handle of the imaging catheter assembly. The control box may also include a wireless transceiver. This wireless transceiver enables the control box to receive wireless video signals from the imaging device and transmit control commands to the imaging device.

The wireless signal transmission and the use of batteries eliminate the need for electrical conductors within the tubular body 36. This reduces the restrictions imposed by electrical conductors to the physician's movements of the endoscope. Additionally, reducing the number of electrical conductors in the catheter and the flex-PCB allows for a larger diameter channel to be included in the catheter.

While the imaging catheter has been described throughout the description as being deployed inside an endoscope, in other applications it may be deployed through other methods such as through a straight tube or cannula, by a flexible insertion tube, or by a guide wire.

## Claims

1. An imaging catheter assembly (20), comprising:
a tubular body (36);
an imaging device (42);
a flexible link (44) that connects the imaging device to the tubular body; and
a light source (45), wherein
the link includes a flexible shape memory element (74) ; **characterized in that** the link includes a flexible PCB (76), and
the light source is placed on said link.

2. The assembly of claim 1, wherein the flexible link (44) has a U-shaped natural configuration including two end segments, wherein one of the end segments is connected to the imaging device and the other end segment is connected to the tubular body.

3. The assembly of claim 2, wherein, in said U-shaped natural configuration, said end segments are substantially parallel to each other.

4. The assembly of claim 2, wherein the flexible link (44) returns to its natural configuration after a force that straightens the flexible link is removed.

5. The assembly of claim 2, wherein the link (44) includes flexible electrical conductors connecting electrical conductors from the imaging device to electrical conductors in the tubular body.

6. The assembly of claim 1, wherein the shape memory element (74) and flexible PCB (76) are placed side by side in a width direction of the flexible PCB.

7. The assembly of claim 1, wherein the shape memory element (74) and flexible PCB (76) are attached to each other.

8. The assembly of claim 1, wherein the shape memory element (74) and flexible PCB (76) are stacked to form a layered structure.

9. The assembly of claim 8, wherein the flexible PCB (74) includes electrical conductors connecting electrical conductors from the imaging device to electrical conductors (78) in the tubular body.

10. The assembly of claim 1, wherein the light source (45) is placed on a curved section of the link (44).

11. The imaging catheter assembly of claim 1, wherein the flexible link (44) may be straightened for insertion into an endoscope and wherein the flexible link resumes its original shape when it is extended beyond the endoscope, the imaging device providing a retrograde view when the flexible link resumes its original shape.

12. An endoscope assembly (10, 110), comprising:
an insertion tube (12) having an end; and
an imaging catheter assembly (20) according to any previous claim.

13. A method of configuring an endoscope (10, 110), comprising:
straightening a U-shaped flexible shape memory link at a distal end of an imaging catheter assembly (20) according to any of claims 1-11;
inserting the straightened distal end of the imaging catheter assembly into a channel of an endoscope's insertion tube (12) from a proximal end of the insertion tube; and
pushing the imaging catheter assembly further into the insertion tube until an auxiliary imaging device (42) and the flexible link (44) are pushed out of the distal end of the insertion tube so that the flexible link returns to its natural bent configuration.

## Patentansprüche

1. Bildkatheteranordnung (20) umfassend:
einen Rohrkörper (36);
ein Bildgebungsgerät (42);
ein flexibles Verbindungsglied (44), welches das Bildgebungsgerät mit dem Rohrkörper verbindet; und
eine Lichtquelle (45), wobei
das Verbindungsglied ein flexibles Formgedächtniselement (74) umfasst; **dadurch gekennzeichnet, dass** das Verbindungsglied eine flexible Leiterplatte (76) umfasst und
die Lichtquelle auf dem Verbindungsglied angeordnet ist.

2. Anordnung nach Anspruch 1, wobei das flexible Verbindungsglied (44) eine ursprüngliche U-förmige Anordnung mit zwei Endsegmenten hat, wobei eines der Endsegmente verbunden mit dem Bildgebungsgerät ist und das andere Endsegment verbunden mit dem Rohrkörper ist.

3. Anordnung nach Anspruch 2, wobei die Endsegmente in der ursprünglichen U-förmigen Anordnung wesentlich parallel zueinander sind.

4. Anordnung nach Anspruch 2, wobei das flexible Verbindungsglied (44) bei Beendigung einer Kraftwirkung, die das flexible Verbindungsglied gerade biegt, wieder in seine Ursprungsform zurückkehrt.

5. Anordnung nach Anspruch 2, wobei das Verbindungsglied (44) flexible elektrische Leiter umfasst, die elektrische Leiter vom Bildgebungsgerät mit im Rohrkörper gelegenen elektrischen Leitern verbinden.

6. Anordnung nach Anspruch 1, wobei das Formgedächtniselement (74) und die flexible Leiterplatte (76) nebeneinander in einer Breiterichtung der flexiblen Leiterplatte angeordnet sind.

7. Anordnung nach Anspruch 1, wobei das Formgedächtniselement (74) und die flexible Leiterplatte (76) aneinander befestigt sind.

8. Anordnung nach Anspruch 1, wobei das Formgedächtniselement (74) und die flexible Leiterplatte (76) zu einer Schichtstruktur gestapelt sind.

9. Anordnung nach Anspruch 8, wobei die flexible Leiterplatte (76) elektrische Leiter umfasst, die elektrische Leiter vom Bildgebungsgerät mit im Rohrkörper gelegenen elektrischen Leitern (78) verbinden.

10. Anordnung nach Anspruch 1, wobei die Lichtquelle (45) auf einem gekrümmten Teil des Verbindungsglieds angeordnet ist (44).

11. Bildkatheteranordnung nach Anspruch 1, wobei das flexible Verbindungsglied (44) gerade gebogen werden kann, um in ein Endoskop eingeführt zu werden und wobei das flexible Verbindungsglied wider in seine Ursprungsform zurückkehrt, wenn es über das Endoskop hinaus ausgestreckt wird, wobei das Bildgebungsgerät einen Rückblick bietet, wenn das flexible Verbindungsglied wieder in seine Ursprungsform zurückkehrt.

12. Eine Endoskopanordnung (10, 110) umfassend:
ein Einsatzrohr (12) mit einem Ende; und
eine Bildkatheteranordnung (20) nach einem der vorhergehenden Ansprüche.

13. Verfahren zur Anordnung eines Endoskops (10, 110) umfassend die folgenden Schritte:
das Geradebiegen eines U-förmigen flexiblen Formgedächtnisverbindungsglieds an einem distalen Ende einer Bildkatheteranordnung (20) nach einem der Ansprüche 1-11;
die Einführung des geradegebogenen distalen Endes der Bildkatheteranordnung in den Kanal eines Einsatzrohrs (12) des Endoskops aus einem proximalen Ende des Einsatzrohrs; und
das Schieben der Bildkatheteranordnung weiter in das Einsatzrohr bis ein Hilfsbildgebungsgerät (42) und das flexible Verbindungsglied (44) aus dem distalen Ende des Einsatzrohres verschoben werden, so dass das flexible Verbindungsglied wieder in seine ursprüngliche gekrümmte Anordnung zurückkehrt.

## Revendications

1. Ensemble (20) de cathéter d'imagerie comprenant:
un corps tubulaire (36);
un dispositif d'imagerie (42);
un lien (44) souple qui raccorde le dispositif d'imagerie au corps tubulaire; et
une source de lumière (45), dans lequel
le lien comprend un élément (74) souple à mémoire de forme ; **caractérisé en ce que** le lien comprend un circuit imprimé (76) souple et que la source de lumière est placée sur ledit lien souple.

2. Ensemble selon la revendication 1, dans lequel le lien (44) souple a une configuration naturelle en forme de U comprenant deux segments finals, dans lequel un des segments finals est raccordé au dispositif d'imagerie et l'autre segment final est raccordé au corps tubulaire.

3. Ensemble selon la revendication 2, dans lequel lesdits segments finals sont essentiellement parallèles l'un par rapport à l'autre dans ladite configuration naturelle en forme de U.

4. Ensemble selon la revendication 2, dans lequel le lien (44) souple revient à sa configuration naturelle dès qu'une force qui redresse le lien souple est supprimée.

5. Ensemble selon la revendication 2, dans lequel le lien (44) comprend des conducteurs électriques souples qui raccordent des conducteurs électriques du dispositif d'imagerie à des conducteurs électriques placés dans le corps tubulaire.

6. Ensemble selon la revendication 1, dans lequel l'élément (74) à mémoire de forme et le circuit imprimé (76) souple sont placés côte à côte dans un sens de la largeur du circuit imprimé souple.

7. Ensemble selon la revendication 1, dans lequel l'élément (74) à mémoire de forme et le circuit imprimé (76) souple sont rattachés l'un à l'autre.

8. Ensemble selon la revendication 1, dans lequel l'élément (74) à mémoire de forme et le circuit imprimé (76) souple sont empilés pour former une structure en couches.

9. Ensemble selon la revendication 8, dans lequel le circuit imprimé (76) souple comprend des conducteurs électriques qui raccordent des conducteurs électriques du dispositif d'imagerie à des conducteurs électriques (78) placés dans le corps tubulaire.

10. Ensemble selon la revendication 1, dans lequel la source de lumière (45) est placée sur une section courbée du lien (44).

11. Ensemble de cathéter d'imagerie selon la revendication 1, dans lequel le lien (44) souple peut être redressé pour son insertion dans un endoscope et dans lequel le lien souple revient à sa forme initiale lorsqu'il est étendu au-delà de l'endoscope, fournissant le dispositif d'imagerie une vue rétrograde lorsque le lien souple revient à sa forme initiale.

12. Ensemble d'endoscope (10, 110) comprenant:
un tube d'insertion (12) ayant un bout; et
un ensemble (20) de cathéter d'imagerie selon l'une quelconque des revendications précédentes.

13. Procédé de configuration d'un endoscope (10, 110) comprenant:
redresser un lien souple à mémoire de forme en forme de U à une extrémité distale d'un ensemble (20) de cathéter d'imagerie selon l'une quelconque des revendications 1à11;
insérer l'extrémité distale redressée de l'ensemble de cathéter d'imagerie dans un canal d'un tube d'insertion (12) de l'endoscope à partir d'une extrémité proximale du tube d'insertion; et
pousser l'ensemble de cathéter d'imagerie plus loin dans le tube d'insertion jusqu'à ce qu'un dispositif d'imagerie (42) auxiliaire et le lien (44) souple soient poussés hors de l'extrémité distale du tube d'insertion de façon à ce que le lien souple revienne à sa configuration courbée naturelle.
